# EUROPÄISCHE PATENTANMELDUNG

(11) **EP 1 908 491 A2**
(43) Veröffentlichungstag der Anmeldung: **09.04.2008**
(21) Anmeldenummer: 07019531.8
(22) Anmeldetag: 04.10.2007
(51) Int. Cl.: A61N 1/32

(54) **Vorrichtung zur Elektroporation und deren Verwendung**

(30) Priorität: 04.10.2006 DE 102006047274
(71) Anmelder: Braun, Michaela, 4731 Eynatten (BE)
(72) Erfinder: Braun, Michaela, 4731 Eynatten (BE)

(57) **Zusammenfassung**

Die Erfindung betrifft eine Vorrichtung zur Elektroporation sowie deren Verwendung:

Erfindungsgemäß werden mit der Vorrichtung Pulse appliziert, welche mindesten einen Öffnungspuls und mindestens einen Erhaltungspuls enthalten. Typischerweise hat der Öffnungspuls eine höhere Spannung als der Erhaltungspuls. Der Öffnungspuls öffnet Hautporen, während der Erhaltungspuls die Dauer der Öffnung der Poren verlängert.

## Beschreibung

Die Erfindung betrifft eine Vorrichtung zur Elektroporation und deren Verwendung.

Bei der Elektroporation werden Substanzen mit dafür vorgesehenen Geräten in die Haut oder in andere Zellen eingebracht.
Das Verfahren wird auch seit den 60er Jahren zur Verschmelzung von Zellen in der Gentechnik angewendet. Weitere Anwendungen sind auch in der Medizin bekannt, um - mit einer bestimmten Elektrodenanordnung - Medikamente gezielt (Menge und Anwendungsort) durch die Haut in den Körper zu bringen. Die Bauart der medizinischen Elektroden ist sehr klein. Diese Elektroden werden festgeklebt.
In der Kosmetik wird mit verschiedenen Pulsformen versucht, Produkte in die Haut zu schleusen. Durch die dort angewendete Pulsform wird jedoch keine Elektroporation erreicht. Zum Einbringen von Substanzen in die Haut wird nach dem Stand der Technik auch die Methode der Mesotherapie eingesetzt, bei der an sehr vielen Hautstellen nebeneinander Wirkpräparate - beispielsweise mittels Spritzen - in die Haut eingebracht werden, die aber sehr schmerzhaft ist, weswegen ein Betäubungsmittel notwendig ist und auch Infektionsgefahr besteht.

Aus der DE 696 24 668 T2 ist eine Vorrichtung bekannt, die ein Einschleusen von Substanzen in die Haut mittels Ultraschall ermöglicht.

Aufgabe der Erfindung ist es, eine Vorrichtung zur Verfügung zu stellen, mit denen Substanzen schmerzfrei in die Haut eingebracht werden können, so dass keine Schmerzmittel mehr verabreicht werden müssen. Insbesondere soll es möglich sein, die Substanzen großflächig in die Haut einzubringen. Gegenüber bestehenden Verfahren und Vorrichtungen soll mehr Substanz in die Haut eingeschleust werden können als beispielsweise bei den kosmetischen Geräten des Standes der Technik.

Die Wirksamkeit soll verbessert werden, wodurch auch die Behandlungszeit verringert werden soll. Die Verwendung soll die genannten Aufgaben ebenfalls lösen.

Erfindungsgemäß wird diese Aufgabe durch eine Vorrichtung nach Anspruch 1 und deren Verwendung mit den Merkmalen des unabhängigen Anspruches 6 gelöst.

Vorteilhafte Weiterbildungen der Vorrichtung ergeben sich aus den Unteransprüchen 2-5.

Es ist zweckmäßig, wenn zusätzlich zu einer bevorzugten Plus-(oder Applikator-) Elektrode noch eine Minus - Elektrode vorhanden ist.

Die Minus-Elektrode schließt den Stromkreis durch den Körper zurück zum Gerät. Die "Elektrode" bedeutet insbesondere die die Applikatorelektrode. Die Applikatorelektrode ist in der Ausführung variabel.

Es können beispielsweise kleine Kugeln (Durchmesser bevorzugt zwischen 0,5 und 2cm) ebenso eingesetzt werden wie auch Rollen größeren Ausmaßes (Durchmesser bevorzugt 1 bis 10 cm, Länge vorzugsweise zwischen 2 und 15 cm).

Die Rollen oder Kugeln könnten auch gewellt sein, um zusätzliche Massageneffekte zu erzielen.

Bevorzugt sind Elektroden, die auch in Doppel- oder Mehrfachform auftreten können.

Bevorzugte Materialien sind vergoldete Metalle oder Karbon.

Die eingesetzten Elektroden können auch zur Hautwiderstandsmessung eingesetzt werden. Dies erleichtert die Steuerung des Geräts.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1:: Den prinzipiellen Aufbau der erfindungsgemäßen Vorrichtung
- Fig. 2:: Eine Spannungs-Zeit Kurve der erfindungsgemäßen Vorrichtung

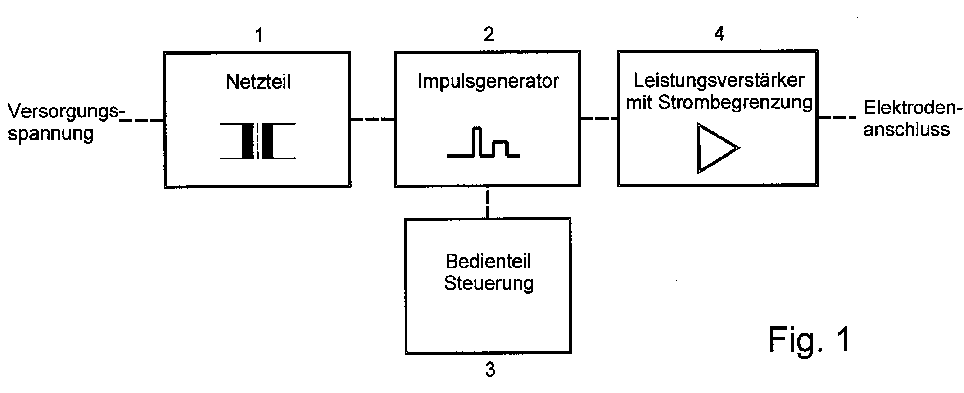

Figur 1 zeigt ein Netzteil 1, welches an einer Spannungsversorgung angeschlossen ist. Das Netzteil 1 steht mit einem Impulsgenerator 2 in Verbindung, welcher an eine Steuerung 3 angeschlossen ist. Der Impulsgenerator 2 steht mit einem Leistungsverstärker 4 mit Strombegrenzung in Verbindung, der in einen Elektrodenanschluss 5 mündet.

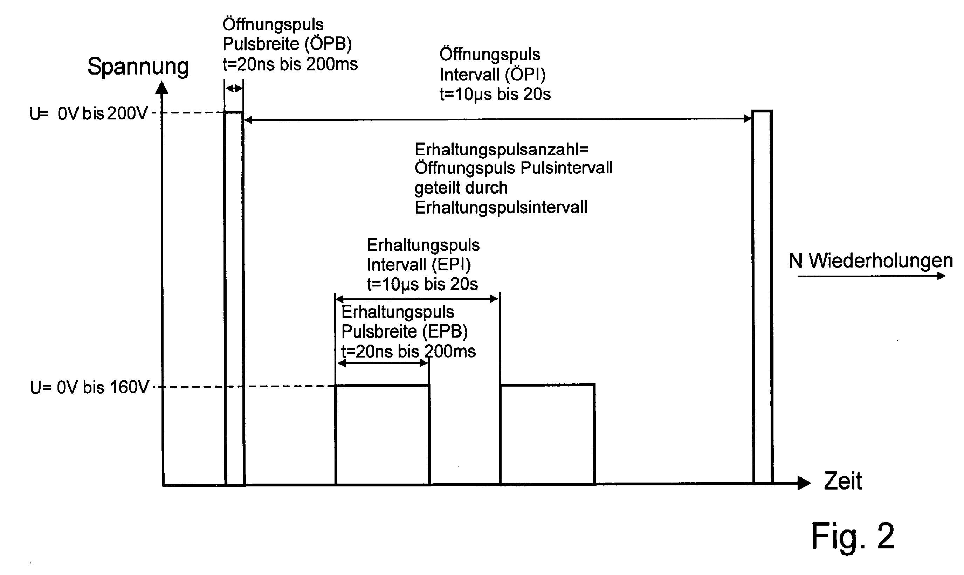

Figur 2 zeigt beispielhaft einen Spannungs-Zeitverlauf. In ihm ist die Ordinate in Volt und die Abszisse in Sekunden angegeben.

Im Folgenden soll die Funktionsweise der erfindungsgemäßen Vorrichtung in ihrer allgemeinen Form beschrieben werden.

Durch die erfindungsgemäße Vorrichtung werden in der Haut Mikroporen geöffnet, die zusätzlich zu den bestehenden Hautporen (Schweißdrüsen und Haarkanäle) eine Durchlassstelle für Wirkstoffe in der Haut bilden. Angewendet wird das Verfahren beispielsweise zur Einschleusung von kosmetischen oder medizinischen Wirkpräparaten in die Haut.

Erfindungsgemäß wird der Wirkungsgrad eines Elektroporationsgerätes erhöht, indem zum Einbringen von Substanzen ein Öffnungspuls appliziert wird, welcher von mindestens einem Erhaltungspuls gefolgt wird.

Mit dem Öffnungspuls werden Poren der Haut geöffnet bzw. geschaffen.

Der Öffnungspuls hat vorzugsweise eine Spannung, die geeignet ist bestehende Poren öffnen und/oder Mikroporen zu schaffen, welche einen Stofftransport in die Haut ermöglichen.

Der Öffnungspuls hat vorzugsweise hohe Spannungswerte in einem Bereich bis ca. 200 V. Typische Werte liegen zwischen 5 V und 220 V, 5 V bis 200 V, 10 V bis 110 V. Besonders bevorzugt sind Bereiche von 20 V bis...80 V oder 15 V bis 80 V. Vorzugsweise beträgt der Öffnungspuls wenigstens 5 V.

Die angewandten Spannungen sollten in Verbindung mit der Stromstärke vorzugsweise zu keiner Schädigung der Haut führen.

Für den Öffnungspuls sind sowohl Gleich- als auch Wechselstrom möglich. Vorzugsweise wird gepulster Gleichstrom eingesetzt. Es kann aber auch gepulster Wechselstrom (z.B. mit kurzen negativen Pulsen) möglich sein. Zur Versorgung des Gerätes kann Gleich- oder Wechselstrom eingesetzt werden.

Die Dauer, also die Pulsbreite des Öffnungspulses ist variabel und kann frei gewählt werden.
Beispielsweise beträgt die Pulsbreite 10 ns bis 250 ms, 20ns bis 200 ms oder auch 50 ns bis 150 ms.

Besonders bevorzugte untere Werte für die Pulsbreite sind zusätzlich zu den genannten Werten von 10 ns, 20 ns und 50 ns: 2 ns und 5 ns. Ebenso wie 0,1 ms.

Besonders bevorzugte obere Werte für die Pulsbreite sind neben den genannten Werten von 150 ms, 200 ms und 250 ms:
Entscheidend ist hier, dass die Dauer - also die Pulsbreite - ausreicht um eine Öffnung der Poren oder Mikroporen zu bewirken.

Bei Betrieb der erfindungsgemäßen Vorrichtung wird mindestens ein Öffnungspuls appliziert. Es ist jedoch auch möglich, wiederholt Öffnungspulse zu applizieren. Die Anzahl der Wiederholungen ist dabei vorzugsweise wählbar. So können beispielsweise 2, 3, 4, oder 5-10 Öffnungspulse appliziert werden, (viel zu wenig) je nachdem, wie viel Substanz in die Haut eingebracht werden soll. Bei einer bevorzugten Behandlungsdauer von etwa 30 Minuten können beispielsweise 9000 Pulse appliziert werden (berechnet bei Pw = 0,2 s).

Das Gerät erzeugt die eingestellten Pulse vorzugsweise zeitlich unbegrenzt. Die Applikationsdauer ergibt sich aus der Zeit, in der sich die Elektroden auf der Haut befinden.

Die Verweildauer jeweils an einer Stelle ist kürzer als die Gesamtdauer.

Dementsprechend ist die Anzahl der Pulse pro Position auf der Haut an einer Stelle geringer als die Gesamtzahl der Pulse.

Die zeitlichen Abstände, in denen die Pulse appliziert werden, werden als Pulsintervall bezeichnet.

Für die Öffnungspulse werden vorzugsweise Pulsweiten Pulsintervalle von 10µs bis 20 s verwendet, jedoch ist auch das Pulsintervall vorzugsweise frei wählbar.

Selbstverständlich können die Pulsintervalle auch andere Dauern mehreren Sekunden, beispielsweise 30s, einer Minute oder mehr annehmen.

Die Pulintervalle für die Öffnungspulse richten sich im Wesentlichen nach der Dauer, für die eine geöffnete Pore offen bleibt. Diese Dauer kann variabel sein.

Die Pulsform des Öffnungspulses ist nicht festgelegt, sondern kann verschiedene Formen annehmen. So ist der klassische Rechteckpuls, eine spitze Pulsform oder auch eine Glockenform, wie beispielsweise einer Gausskurve, möglich.

Um die Wirksamkeit der Elektroporation zu steigern, wird nach dem Öffnungspuls mindestens ein Erhaltungspuls appliziert.

Der Erhaltungspuls hat die Funktion, die einmal geöffnete Pore oder entstandene Mikropore geöffnet zu halten, damit möglichst viel Substanz in die Haut eingebracht werden kann.

Vorzugsweise hat der Erhaltungspuls eine Spannung, die unterhalb derer des Öffnungspulses liegt.

Der untere mögliche Spannungswert ist dadurch gegeben, dass er den Erhalt der Porenöffnung gewährleistet. Er kann bei 3 V liegen. Die Erhaltungspulse können beispielsweise 3 V bis 180 V, 5 V bis 160 V oder 5 V bis 60 V haben.

Die Pulsbreite des Erhaltungspulses ist variabel und kann frei gewählt werden.

Beispielsweise beträgt die Pulsbreite 10 ns bis 250 ms, 20ns bis 200 ms oder auch 50 ns bis 150 ms. Vorzugsweise 0,5 ms bis 20 ms.

Entscheidend ist hier, dass die Dauer ausreicht, um eine Öffnung der Poren oder Mikroporen aufrecht zu erhalten.

Das Pulsintervall für die Erhaltungspulse kann ebenfalls variiert werden. Es kann hier ebenfalls zwischen 10µs bis 20 s liegen, jedoch sind diese Werte nur beispielhaft und nicht beschränkend zu verstehen. Alle Werte, die eine Erhaltung der Öffnung bewirken, können verwendet werden.

Für den Erhaltungspuls sind sowohl Gleich- als auch Wechselstrom möglich. Gepulster Gleichstrom ist jedoch besonders bevorzugt.

Die Pulsform des Erhaltungspulses ist nicht festgelegt, sondern kann verschiedene Formen annehmen. So ist der klassische Rechteckpuls, eine spitze Pulsform oder auch eine Glockenform, wie beispielsweise einer Gausskurve, möglich.

Die Erhaltungspulse können beliebig oft wiederholt werden.

Sowohl Öffnungs- als auch Erhaltungspulse können bezüglich ihrer Pulshöhe variiert werden.

Bei den jeweiligen Spannungen kann Strom fließen. Die Stromstärken sollten so niedrig sein, dass keine Schädigung der Haut eintritt. Vorzugsweise werden geringe Stromstärken appliziert. Vorzugsweise sollten die Stromstärken die gesetzlich zugelassenen Grenzen nicht überschreiten. Diese liegen derzeit bei 200 mA (30 mA bei Dauerstrom). Es sind beispielhaft Stromstärken zwischen Mindestens 0,1 mA, vorzugsweise zwischen 1 mA und 200 mA.

In einer besonders bevorzugten Ausführungsform der Erfindung ergibt sich die Stromstärke aus dem Hautwiderstand und der eingestellten Spannung.

Hierbei ist es vorteilhaft, den elektrischen Widerstand der Haut zu messen. Dies kann beispielsweise auch durch die für die Stromapplikation eingesetzten Elektroden erfolgen.

Insbesondere ist es vorteilhaft, die Spannung vorzugeben, so dass sich die Stromstärke durch den Hautwiderstand ergibt.

Die Erfindung umfasst sowohl Ausführungsformen, bei denen die Spannung zwischen 0 V und maximalen Spannungswerten - bis zu 200 V - variiert als auch Ausführungsformen, bei denen die Spannung zwischen einem unteren Schwellwert - vorzugsweise 5 V - 20 V - und dem maximalen Spannungswert variiert.

Besonders bevorzugte Werte sind 3 mA und 200 mA. Jedoch sind auch Stromstärken zwischen 5, 10, 20, 50 mA und 100m 120, 150, 180 und 200 mA möglich.

Die genannten Variationen von Öffnungs- und Erhaltungspulsen können über die Steuerung 3 ermöglicht werden. Hierbei können fest vorprogrammierte Pulsfolgen und/oder Spannungswerte in Form von Applikationsprogrammen aktiviert werden. Weiterhin ist es möglich die genannten Parameter über ein Wählelement selber in ihrer gewünschten Kombination vorzugeben.

Die verwendeten Elektroden können verschieden ausgestaltet und je nach Anwendungsfall auch ausgetauscht werden. So können spitze Elektroden für punktuelle Anwendungen oder Elektrodenflächen verschiedener Abmessungen für flächige Anwendungen eingesetzt werden, um größere Hautregionen zu behandeln.

Die erfindungsgemäße Vorrichtung ist insbesondere geeignet, pharmakologisch wirksame Substanzen oder Kosmetika in die Haut einzubringen. Beispielsweise aber nicht beschränkend können die Substanzen - wie beispielsweise Insulin, Vitamine, Mineralstoffe, Hyaluronsäure, Schmerzmittel, Immunglobuline, Eiweißstoffe, Antifaltensubstanzen - in die Haut eingebracht werden.

Das Verfahren zum Einbringen sowie die erfindungsgemäße Vorrichtung können durch weitere Methoden ergänzt werden, die weitere physiologische Wirkungen besitzen. Diese sind beispielhaft:

Ultraschall, IR, UV, Variation der Elektrodenbauform (auch in Kombination mit IR/ UV/ Ultraschall o.a. Lichtfrequenzen), Handgerät, Wärme, Saugeffekt / Massage, Magnetfelder, Vibration, Pulsform wechseln lassen.

Variationen der Erhaltungspulsintervalle (EPI) erfolgen vorzugsweise innerhalb eines Öffnungspulsintervalls (ÖPI).

Dies geschieht insbesondere durch eine Veränderung der Spannungswerthöhe und/oder der Pulsbreite.

Es ist vorteilhaft, diese Veränderungen alternierend durchzuführen.

Bei eine vorteilhaften Reizstrombehandlung werden Elektroden über Kreuz großflächig angelegt.

In einer besonders bevorzugten Ausführungsform wird das aufzutragende Produkt auf die Elektroden und/oder die zu behandelnden Hautpartien aufgetragen.

Die Elektroden können verschiedene Bauform aufweisen.

Eine Kombination der dargestellten Maßnahmen mit anderen physikalischen Maßnahmen zur Haut- und/oder Muskelbehandlung, ist vorteilhaft. Insbesondere ist eine Kombination mit Reizstromgeräten wie Slendertone- oder Ultratone-Gerät zweckmäßig.

Insbesondere betriff die Erfindung ein Gerät zum Einschleusen von Produkten in die Haut mittels Elektroporation. Durch die Elektroporation werden in der Haut Mikroporen geöffnet, die zusätzlich zu den bestehenden Hautporen (Schweißdrüsen und Haarkanäle) eine Durchlassstelle für Wirkstoffe in der Haut bilden. Angewendet wird das Verfahren zur Einschleusung von (kosmetischen oder medizinischen) Wirkpräparaten in die Haut. Diese Methode soll die bisher angewendete, schmerzhafte Methode der Mesotherapie ersetzen. Mesotherapie bedeutet: mit Spritzen an sehr vielen Hautstellen nebeneinander Wirkpräparate in die Haut zu bringen. Hierfür ist ein Betäubungsmittel notwendig. Der Vorteil der Elektroporation ist, dass dies nicht mehr notwendig ist, weil die Methode völlig schmerzfrei arbeitet.

Elektroporese (auch Elektroporation genannt) ist ein bestimmtes Verfahren, dadurch gekennzeichnet, dass es erfindungsgemäß eine bestimmte Pulsform a) und Spannungswerte aufweist.
a) Pulsform
   Anwendung: Strom mit der bestimmten Pulsform auf den Körper (die Haut) bringen in Zusammenhang mit kosmetischen oder medizinischen Produkten mittels Elektroden.
   • Annähernd Rechteckpulsform (Variante: Spitze ansteigend oder abfallend)
   • Öffnungs- und Erhaltungs-(=Folge-)pulse
   • 0 - 200 V für Öffnungspuls
   • Gleich- oder Wechselstrom möglich Pulsweitenregulierung (von 10 µs bis 20 s)
   • Variable Pulsbreite: von 20 ns bis 200 ms (Öffnungspuls) bzw. 20 ns bis 200 ms (Erhaltungspuls)
   • Erhaltungspulse 0 - 160 V
   • Beliebig viele Wiederholungen
   • (z.B. alternierende Pulshöhenvariation der Erhaltungspulse auch möglich: Höhe, Breite oder Abstände der Erhaltungspulse variieren innerhalb eines Intervalls)
   • Intervallbreite von 0,1 µs bis 20 s.
b) Gerät
   Geräteaufbau:

Siehe Zeichnung
   Regulierbare Pulse, variable Einstellmöglichkeiten.
   Ferner können feste Vorwahlgrößen eingesetzt werden.
Die Anzahl der Kanäle ist beliebig.
   Ebenso können verschiedene Elektrodenarten eingesetzt werden.

### Vorteile des beschriebenen Verfahrens:

Bessere Wirksamkeit in Vergleichstests: es kann mehr Produkt in die Haut eingeschleust werden als bei den beschriebenen kosmetischen Geräten.

Über die gebildeten Elektroporen gehen die Produkte/Wirkstoffe in die Haut. Es kann großflächig angewendet werden.

Bevorzugt sind Elektroden der Polarität + und -. Eine dieser Elektroden dient als Applikationselektrode, die andere als Ableitelektrode. Die Applikationselektrode ist bevorzugt als +Pol zu sehen und ist elektrisch leitfähig, den -Pol bildet z.B. eine leitfähig beschichtete Gummi- oder Karbonelektrode. Es ist je nach Anwendung möglich, die Polung der Elektroden zu tauschen.

Der Aufbau der Elektroden ist dabei variabel. Bevorzugt wird eine vergoldete Applikationselektrode.

Es können mehrere Applikationselektroden (Variation in Größe und Bauform) pro Gerät verwendet werden. Mehrere Kanäle am Gerät sind möglich (z.B. Unterscheidung verschiedener Hautareale durch verschiedene Voreinstellungen).

### Varianten:

Zusätzliche Erweiterung des Gerätes bzw. der Elektroden durch einzelne oder eine Kombination der folgenden Zusätze:
1. Ultraschall
2. IR
3. UV
4. Variation der Elektrodenbauform (auch in Kombination mit IR/ UV/ Ultraschall)
5. Handgerät
6. Wärme
7. Saugeffekt / Massage
8.Magnetfelder
9. Vibration
10. Pulsform wechseln
11. Vergleich Reizstrombehandlung: Elektroden über Kreuz großflächig anlegen
12. Elektrode mit Produkt kombinieren - andere Bauform
13. Kombination mit anderen physikalischen Maßnahmen zur Muskelbehandlung, vgl. Ultratone-Gerät
14. Zusätzlicher Einsatz von Hochfrequenzbestrahlung.

### Produktspezifikationen:

Bevorzugt werden elektrisch leitende Produkte appliziert. Dies sind beispielhaft Salz oder Ionen enthaltende Stoffe (die Polarität ist egal).

### Bevorzugte Produktspezifikationen:

Eher etwas dünnflüssiger, unkonserviert bzw. sehr niedrige Dosierung des Konservierungsmittels (bevorzugt Sterilabfüllung), hoher Wirkstoffgehalt.

Stoffe, die nicht in die Haut gehören (z.B. Fruchtsäuren, ätherische Öle, Emulgatoren, fette Öle, leicht ranzig werdende Substanzen, Keime, Schimmelpilze usw.) sind vorzugsweise zu vermeiden.

Elektronische Geräte zur kosmetischen und/oder medizinischen Behandlung der Haut mit (Wirk)produkten.

Wenn man Salzwasser einschleust, lässt sich die Hautfeuchtigkeit erhöhen.

Elektronische Geräte zur Hautbehandlung bedienen sich bisher der Menge Hautporen, die durch Schweißdrüsen, Haarkanäle usw. biologisch bedingt vorliegen. Vgl. Iontophorese.

Durch eine geeignete elektrische Pulsform des Stromes ist es gelungen, in der Haut (für Sekundenbruchteile) so genannte Mikroporen zu öffnen und diese für die Dauer der Behandlung auch geöffnet zu halten (solange Strom auf die Haut gebracht wird. Bevorzugt wird mittels Elektroden die Hautstelle nacheinander bearbeitet, und die Stelle dann mit Wirkstoffen behandelt, d.h. Areale werden nacheinander bearbeitet.

Dadurch kann ein Mehrfaches des Wirkproduktes aufgetragen und eingeschleust werden als bei einer Anwendung ohne Strom oder mit einer anderen Pulsform.

Eine Vorbehandlung der Haut durch geeignete Reinigungsmaßnahmen ist sinnvoll, um die Einschleusung zu erhöhen, z. B. für Fruchtsäurepeeling.

### Bevorzugte Ausführungsformen sind:

1. Lange Pulse: als Öffnungspulse (hohe Spannungswerte, geringe Pulsbreite)
2. Folgepulse (heißen auch Erhaltungspulae): geringere Spannungswerte, Pulsbreite variabel, sind bevorzugt aber nicht zwingend breiter als die Öffnungspulse, beliebig viele Wiederholungen, bis der nächste Öffnungspuls kommt.
3. Nr. 1 (Öffnungspuls und Nr. 2 als Wiederholung (bis zum Ende der Behandlung)
4. Intervallweite von 20 ns bis 20 s (siehe Zeichnung und Beschreibung Seite 1)
5. Pulsform siehe Zeichnung. Die dort angegebenen Intervalle sind variabel.
6. Je nach Körperregion, die zu behandeln ist, sind unterschiedliche Werte einstellbar.

Bei empfindlicheren Regionen wie dem Gesicht werden weniger hohe Spannung und Pulsbreiten eingesetzt.

Durch die Pulsform und den elektrischen Kontakt bilden sich Poren in der Haut, die solange aufrechterhalten werden, wie der elektrische Kontakt besteht. Das anzuwendende Produkt kann an viel mehr Stellen (Poren) in die Haut einziehen, und man kann mehr Produkt einarbeiten.

Im Vergleich zu äußerlich angewendeten Kosmetika, die nur zu sehr geringem Anteil (falls überhaupt) die Hornschranke der Haut durchdringen (z. B. Nanopartikel), hat man hier eine viel bessere und gezieltere Behandlungsmöglichkeit am Ort des Hautproblems. Dies ermöglicht eine gezielte Auftragung bei Falten oder Cellulite. In Verbindung mit den geeigneten Wirkstoffen können Hautprobleme gezielter behandelt werden.

Nachfolgend werden Parameter für eine besonders bevorzugte Ausführungsform der Erfindung genannt:
Eine bevorzugte Untergrenze Öffnungspuls ist 5 Volt. Die Obergrenze ist 110 Volt.

Erhaltungspuls: ab 3 Volt (ca. halb so hoch wie der Öffnungspuls) maximal 60 Volt (Schmerzempfindlichkeit geringer) Spannung.

### Pulsbreiten:

Der Öffnungspuls (Öp) wird schmaler gemacht als der Erhaltungspuls.
Öp unter 20 Mikrosekunden (µs) - 100 µs - 120 µs oder mehr:
0,1 - 0,5 - unter 1,5 Mikrosekunden möglich.

Der Erhaltungspuls (Ep) weist vorzugsweise folgende Werte auf:
P 0,01 ms - 0,5 ms bis 3 ms - 5 ms-
   Sollte weit unter 10 ms liegen, da es sonst zu intensiv wird. 0,3 ms (=300 µs)

Besonders eignet sich die Erfindung für ein Auftragen von Medikamenten und Kosmetika.

Dies sind insbesondere Hormone (z.B. Östrogen, Testosteron) Cortison, Aminosäuren (z.B. Interferon, Immunglobuline, Immunsubstanzen, Wachstumsfaktoren, Blutdruckmittel, Insulin, Homööpathika, (bevorzugt auch Wirkstoffe, die gezielt an der Stelle aufgetragen werden, wo sie wirken sollen)

Vitamine, Mineralien, Isoflavone, hauteigene Stoffe (Ceramide o.a), Antifaltensubstanzen, Aknemittel, Haarwuchsmittel, Enzym-Hemmer, Substanzen, die den Abbau von Enzymen blockieren, kosmetische Peptide, Hyaluronsäure.

Bevorzugt sind wässerige Phase aber auch einige Lipide, die denen der Haut entsprechen.

Es ist zweckmäßig, verschiedene Kanäle am Gerät vorzusehen:
Es werden z.B. verschiedene Vorwahlgrößen angegeben, die auf bestimmte Körperregionen abgestimmt sind. So ist z.B. ein Kanal für die empfindlichere Gesichtsregion mit niedrigeren Werten (Spannung und Pulsbreiten) und ein weiterer für die Körperregion möglich. Sinnvoll sind auch fest voreingestellte Programme für die bestimmten Applikationen. Dabei sind - nach Problembereichen unterteilt - mehrere Programme sinnvoll. Die jeweilig zur Anwendung passende Elektrode ist zum Kanal dazugehörig, kann aber auch ausgetauscht werden.

Es könnte auch ein Vorwiderstand die Stromgrenze verringern bei einem Kanal.

### Bevorzugte Elektrodenformen sind:

Roller-Elektroden verschiedener Größe: Z. B. Metallrollen, die z.B. 3 cm lang und 1-2 cm Durchmesser oder z.B. 15 cm lang mit 2-6 cm Durchmesser für Körperanwendung (Oberschenkel) sein können. Die Rollen befinden sich vereinigt in einem Handgriff und sind je einzeln an das Gerät angeschlossen. Eine Rolle kann auch integrierte Massageelemente besitzen (wie Wellenform o.ä.)

Weitere Varianten können zusätzliche Funktionen - integriert in die Elektrode - sein: UV- oder IR-Licht, zusätzliche Hochfrequenzbestrahlung, Ultraschallerzeugung im Elektrodengriff (separat oder zusätzlich zum UV-/ IR-Gerät

Für das Gesicht (Augenpartie) ist insbesondere eine Kugelelektrode geeignet. Sie kann auch doppelte Form haben (zwei Kugeln parallel), die zusammen an einem Handgriff sitzen. Gute Abmessungen für Kugeln sind beispielsweise um 1 cm Durchmesser.

Auch größere Flachelektroden oder Flächenelektroden können angewendet werden.

Es können auch verschiedene Formen von Applikatoren zu einer Handelektrode zusammengefasst werden. (z.B. Rolle und Kugel in einer Elektrode vereint), um nicht immer umstecken zu müssen. Durch Drehung kann die eine oder andere Form direkt angewendet werden.

Als weitere Variante ist eine in die Elektroden integrierte Hautwiderstandsmessung sinnvoll. Mit Gleichstrom an der Elektrode angelegt, kann der Hautwiderstand gemessen werden.

Die Produkte werden direkt auf die Haut aufgetragen. Es kann auch eine Elektrodenform gewählt werden, die das Produkt in einem in die Elektrode integrierten Behältnis beherbergt und dieses dann während der Anwendung freigibt. Das Produkt muss aber dann elektrischen Kontakt herstellen können.

Zur Schließung des Stromkreises wird zusätzlich zur. Applikationselektrode auch eine Gegenelektrode benötigt. Diese kann aus einer leitfähig beschichteten Gummielektrode oder auch einer Karbonelektrode oder einer festgeklebten Patch-Elektrode bestehen. Die Größe sollte z.B. mindestens 1x 1 cm, besser 4x4, 4x6 oder 10 x 10 cm betragen., um eine Ableitung zu gewährleisten. Die Gegenelektrode kann rund oder eckig sein oder eine beliebige Form besitzen. Bevorzugt ist diese flach. Die Elektrode wird an den Körper angelegt, wobei sie während der Behandlung einer Körperregion nicht mehr bewegt wird. Sie sollte mit Salzwasserlösung oder leitfähigem Produkt benetzt werden, um die Leitfähigkeit zu erhöhen.

Wechselwirkung von Produkt und Elektroden:
1. das Produkt wird bevorzugt vor der Anwendung auf die Haut aufgetragen (in größerer Menge). Mit den Elektroden wird solange auf der anzuwendenden Hautpartie hin und hergefahren, bis das Produkt in der Haut aufgenommen ist.
2. Problematisch ist durch den Elektrodenfluss bedingte Oxidation des Produktes (Oxidation heißt ja bekanntlich Elektronenzunahme, was zu Bildung von Peroxiden führen könnte), weshalb ein Oxidationsmittel zuzusetzen ist, bzw. nur Produkte angewendet werden dürfen, die gegen Oxidation geschützt sind (Zusatz geeigneter Antioxidanzien, z.B. Vitamin E oder andere).
3. Die Elektroden bewirken durch den Kontakt der gepulsten Spannung mit der Haut eine Öffnung von Poren (so genannten Elektroporen) zusätzlich zu bestehenden Hautporen. Das Produkt dringt dann in die Haut ein und wird dort aufgenommen. Das heißt, es wird gezielt an den notwendigen Stellen das für ein Hautproblem richtige Produkt aufgetragen. Es ist sinnvoll, nur jeweils relativ kleine Hautareale intensiv zu bearbeiten und dann erst weitere, da ansonsten der Abstand zu groß wird und die neu gebildeten Poren sich wieder schließen könnten.

### Angabe der Zeitdauern:

1. Behandlungsdauer: Gesamtzeit zwischen 5 Minuten bis auch 45 oder 60 Minuten (je nach Körperregion), Werden nacheinander verschiedene Körperzonen behandelt, so summiert sich das zu einer noch größeren Gesamtzeit. Es werden verschiedene Hautareale mit Produkt benetzt. Bei dieser Anwendungsform ist mehr Produkt nötig, als durch einfaches Einreiben von der Haut aufgenommen werden kann. Dann wird das Produkt z.B. auf einer Fläche von 5 x 5 cm oder 10 x 10 cm in die Haut eingearbeitet (dies dauert gewöhnlich einige Zeit, bis das Produkt plötzlich in der Haut verschwindet). Danach wird dann das nächste Hautareal benetzt usw. Insoweit ist die Gesamtzeit der Applikation unabhängig von der Dauer des Gerätebetriebes zu sehen. Für die Einarbeitung von Produkt auf den genannten kleinen Flächen ist mit der entsprechenden Anzahl Pulse eine gewisse Zeit notwendig.
2. Das Gerät gibt kontinuierlich die eingestellten Pulse aus, jedoch wird die Applikationselektrode nicht unbedingt ununterbrochen auf der Haut verweilen. Die Anzahl der Öffnungspulse ist gewöhnlich höher als angegeben, weil die Pulse auf die gesamte Haut verteilt werden, abhängig von der Bewegung. Die Elektrode soll nicht längere Zeit auf der gleichen Stelle verweilen, da es sonst zu Verbrennungen kommen kann.
3. Die Pulsbreite soll so gering wie möglich eingestellt werden. Öffnungspulse sind deutlich schmaler als die Erhaltungspulse (Pulsbreite).

Die Anzahl der Pulse über die Zeitdauer variiert mit der Einstellung. Beispielhaft einige Werte zur Verdeutlichung:
Bei einer Anwendung von 2 s als Intervall bedeutet das: alle 2 s kommt ein Öffnungspuls und dazwischen kommen z.B. ca. 20 Erhaltungspulse (abhängig von der Einstellung der Erhaltungspulse). Das läuft über die gesamte Behandlungszeit. Bei einer Anwendungsdauer von 30 Minuten (z.B. ganzes Gesicht) bedeutet das dann entsprechend viele Pulse: bei 10 Minuten Anwendungsdauer werden 300 Öffnungspulse, bei 30 Minuten 900 Öffnungspulse appliziert.

Die Applikations-Elektrode ist nicht fest auf der Haut befestigt und liegt nicht dauernd auf der Haut an, sondern wird bewegt. Hiervon ist die Anzahl der Pulse abhängig. (Die Gegenelektrode ist fest befestigt und dient zur möglichst großflächigen Körperkontaktierung.) Es kann sowohl die Gegenelektrode öder auch die Applikationselektrode der positive oder negative Pol sein. Die andere weist jeweils dann die umgekehrte Polung auf. Bevorzugt wird für kosmetische Anwendung die Applikationselektrode als positiver Pol eingesetzt. Je nach aufzutragendem Produkt kann es sinnvoll sein, die Polung zu ändern.

Die Gegenelektrode, die bevorzugt an der gegenüberliegenden Seite der zu behandelnden Hautpartie angebracht wird, ist für Erhöhung der Leitfähigkeit mit Wasser oder Salzwasser oder Produkt zu benetzen, um den Widerstand zu verringern.

Es werden die Werte für die Pulsbreiten von Öffnungs- und Erhaltungspulsen, die Intervalle und die jeweiligen Spannungswerte eingestellt. Die Häufigkeit der Erhaltungspulse bis zum nächsten Öffnungspuls berechnet das Gerät intern selbst aus den eingestellten Zeiten.

### Messung der Pulsform:

1. Die Pulsform wird ohne Last mit einem Oszilloskop gemessen, d.h. nicht an der Haut, sondern direkt am Ausgang des Gerätes. Das sind die Werte, die hier immer angegeben werden. Das sind die im Gerät eingestellten Werte und Pulse.
2. An der Haut: mittels der Elektrode und dem aufgetragenen Produkt kann man die real applizierte Pulsform messen, die je nach Hauttyp des Patienten trotz der immer gleichen Einstellungen am Gerät stark variieren kann. Die eingestellten Abstände bleiben konstant, jedoch kann sich eine Rechteckform zu einer anderen Pulsform verändern oder auch gehen die gemessenen Spannungswerte unter die eingestellten Werte.

Eine Grundspannung kann ebenfalls variiert werden. Bevorzugt gehen die Pulse auf Null Volt zurück, es kann aber eine Grundspannung (z.B. 10 V) eingestellt werden, die ggf. auch negativ sein kann.

Grundsätzlich sind positive oder negative Pulse möglich. Bevorzugt wird hier die positive Pulsform.

Am Eingang des Gerätes kann sowohl Gleich- als auch Wechselstrom betrieben werden. Am Ausgang des Gerätes befindet sich immer die jeweilige eingestellte Pulsform.

Nach einem Öffnungspuls erfolgt eine Pause, dann eine verschiedene Anzahl von Erhaltungspulsen mit jeweiligen Pausen dazwischen bis zum nächsten Öffnungspuls.

Pause bedeutet: Null Volt (falls keine Vorspannung eingestellt ist)

Bevorzugte Stromstärken liegen bei Milliampere, Mikroampere, nanoAmpere. Der Strom ergibt sich aus dem Hautwiderstand nach dem Ohmschen Gesetz und wird vorzugsweise nicht vorgegeben.

### Bezugszeichenliste:

- 1: Netzteil
- 2: Impulsgenerator
- 3: Steuerung
- 4: Leistungsverstärker
- 5: Elektrodenanschluss

## Patentansprüche

1. Vorrichtung zur Elekroporation umfassend eine Spannungsquelle und mindestens eine Elektrode zur Applikation von Strompulsen,
**dadurch gekennzeichnet,**
**dass** sie Mittel zur Applikation von Strompulsen besitzt, welche mindestens einen Öffnungspuls und mindestens einen Erhaltungspuls erzeugen, wobei der Öffnungspuls die Wirkung hat, Poren und/oder Mikroporen zu öffnen und der Erhaltungspuls die Wirkung hat die Poren und/oder Mikroporen offen zu halten.

2. Vorrichtung nach Anspruch 1,
**dadurch gekennzeichnet,**
**dass** sie eine Applikationselektrode enthält, die aus einem vergoldeten Metall besteht.

3. Vorrichtung nach Anspruch 1,
**dadurch gekennzeichnet,**
**dass** die Elektrode aus Karbon besteht.

4. Vorrichtung nach einem der Ansprüche 1 bis 3,
**dadurch gekennzeichnet,**
**dass** die mit einem Reservoir für die Aufnahme des aufzutragenden Produktes verbunden ist..

5. Vorrichtung nach einem der Ansprüche 1 bis 4,
**dadurch gekennzeichnet,**
**dass** die eingesetzten Elektroden zur Hautwiderstandsmessung eingesetzt werden.

6. Verwendung einer Vorrichtung nach einem der Ansprüche 1 bis 5,
**dadurch gekennzeichnet,**
**dass** die Vorrichtung zum Einschleusen von Produkten in die Haut eingesetzt wird.
